(19) Europäisches Patentamt
European Patent Office
Office européen des brevets

(11) EP 2 737 916 B1

(12) **EUROPEAN PATENT SPECIFICATION**

(45) Date of publication and mention of the grant of the patent:
**27.05.2020 Bulletin 2020/22**

(21) Application number: **12816875.4**

(22) Date of filing: **20.06.2012**

(51) Int Cl.:
***A61M 1/16*** *(2006.01)* ***B01D 61/28*** *(2006.01)*
***B01D 69/08*** *(2006.01)* ***B01D 71/38*** *(2006.01)*
***B01D 71/48*** *(2006.01)* ***B01D 71/44*** *(2006.01)*
***B01D 71/68*** *(2006.01)* ***D01F 6/00*** *(2006.01)*
***D01F 6/76*** *(2006.01)* ***D01F 6/94*** *(2006.01)*
***B01D 67/00*** *(2006.01)* ***B01D 65/02*** *(2006.01)*
***B01D 69/02*** *(2006.01)*

(86) International application number:
**PCT/JP2012/065737**

(87) International publication number:
**WO 2013/015046 (31.01.2013 Gazette 2013/05)**

(54) **HOLLOW FIBER MEMBRANE BLOOD PURIFICATION DEVICE**
BLUTREINIGER VOM HOHLFASERMEMBRANTYP
ÉPURATEUR DE SANG DU TYPE MEMBRANE CAPILLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2011 JP 2011164788**

(43) Date of publication of application:
**04.06.2014 Bulletin 2014/23**

(73) Proprietor: **Asahi Kasei Medical Co., Ltd.**
**Tokyo 100-0006 (JP)**

(72) Inventors:
- **SATOH, Junya**
**Tokyo 101-8101 (JP)**
- **SEKINE, Kumiko**
**Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**EP-A1- 2 151 273** **EP-A1- 2 286 902**
**WO-A1-2009/123088** **GB-A- 1 415 597**
**JP-A- 2009 022 635** **JP-A- 2010 104 984**
**JP-B2- 4 190 079** **JP-B2- 4 325 904**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 737 916 B1

## Description

### Technical Field

The present invention relates to a hollow-fiber membrane blood purification device.

### Background Art

Conventionally, hollow-fiber membrane blood processing devices using a permselective membrane have been widely used in the field of extracorporeal blood circulation, for example, hemodialysis, oxygen supply to blood during open-heart surgery or plasma separation.

Recently, in the field of blood processing membranes, in particular, dialysis membranes, gas exchange membranes and blood component separation membranes, a hollow-fiber membrane for processing blood (hereinafter, sometimes simply referred to as a hollow-fiber membrane) composed of a polysulfone-based resin (hereinafter, sometimes referred to as a polysulfone) has been widely used.

If a highly hydrophobic polysulfone alone is used as a constitutional material for the hollow-fiber membrane, sufficient compatibility with blood cannot be obtained. For this reason, a complex of a polysulfone with a hydrophilic polymer such as polyvinylpyrrolidone, is generally used.

In an attempt to make a hollow fiber membrane to play a role not only as a separation membrane but also in mitigating oxidative stress, which is obviously seen in patients receiving dialysis for a long time, some trial studies have been made. Examples of the studies may include eliminating a causative substance of oxidative stress, peroxides, and regaining an antioxidative effect of organisms by use of hollow-fiber membranes. More specifically, a hollow-fiber membrane blood purification device provided with a hollow-fiber membrane (used as a dialysis membrane) fixed with vitamin E (a fat-soluble vitamin) having various physiological effects such as an *in vivo* antioxidative effect, a biomembrane stabilizing effect and a platelet aggregation inhibitory effect has been proposed (see, e.g., Patent Document 1).

From clinical studies, it has been reported to reduce the dose of a hematopoietic agent, which is indispensable for dialysis treatment but extremely expensive, by use of a hollow-fiber membrane blood purification device provided with a hollow-fiber membrane with vitamin E fixed thereto (see, e.g., Non-Patent Documents 1 and 2). Information of these reports suggests that wide use of a fat-soluble vitamin (specifically, vitamin E) fixed hollow-fiber membrane blood purification device is desired from a medical economic point of view.

It has also been reported (see, e.g., Patent Document 2) that if the hollow-fiber membrane blood purification device provided with a hollow-fiber membrane with a fat-soluble vitamin fixed thereto is used in dialysis treatment, the membrane serves to prevent invasion of a pyogenic substance (endotoxin), which may get in from a dialysis fluid. The properties of a hollow-fiber membrane with a fat-soluble vitamin fixed thereto are considered to be extremely useful for dialysis treatment, performed with a dialysis fluid having a low cleanliness level, presumably in a region where a blood purification treatment has not been so far made.

In the meantime, the hollow-fiber membrane blood purification devices as mentioned above are roughly divided into a wet-type device, in which the interior hollow-portion of a hollow-fiber membrane and the clearance with a container are filled with an aqueous medium, and a dry-type device not filled with an aqueous medium.

The dry type devices are further divided into a dry type of a narrow sense, in which the moisture content of a hollow-fiber membrane is several percent or less, and a dry type in which a hollow-fiber membrane is appropriately moisturized with e.g., water or a humectant. The latter one is sometimes called as a semi-dry type distinguishably from the dry type of a narrow sense; however, since they have almost the same properties, they will be collectively referred to as a dry type in this specification.

Since the dry type devices are light compared to wet type devices and the interior liquid will not vigorously move even if impact is given, the hollow-fiber membrane is rarely broken. Besides this, they are rarely frozen at low temperature. Because of these features, the dry type devices are superior in view of distribution such as transportation and storage. These properties are extremely useful for expanding the devices to the region where blood purification treatment has not been carried out and a delicate aspect in distribution of a hollow-fiber membrane blood purification device has not been understood.

### List of Prior Art Documents

#### Patent Document

Patent Document 1: Japanese Patent Laid-Open No. H7-178166

Patent Document 2: Japanese Patent Laid-Open No. 2009-22635

Non-Patent Document

Non-Patent Document 1: Andrulli S et.al., Nephron Clin Pract. 2010, 115: c82-89
Non-Patent Document 2: Panichi V et al., Blood Purification. 2011 32: 7-14

EP 2151273 discloses hollow-fiber membranes for blood treatment, said hollow-fiber membranes having a long term stability. The hollow-fiber membrane comprises a polysulfone-based resin, a polyvinyl pyrrolidone, a fat-soluble vitamin E and is characterized by an abundance of the fat-soluble vitamin on all surfaces of the hollow-fiber membrane of 4 mg or more and 25 mg or less based on 1 g of the hollow-fiber membrane. The hollow-fiber membrane is in dry state.

EP 2286902 discloses a hollow-fiber membrane comprising a polyester on its surface having an improved compatibility with blood and resistance to the deposition of proteins or organic substances. The polymer includes poly hydroxyethyl methacrylate. The presence of antioxidants such as vitamin C is also disclosed.

JP 4-190079 discloses a hollow-fiber membrane for hematocatharsis and a hollow-fiber membrane type artificial kidney in which endotoxine is adsorbed on an outer surface and to provide a hollow-fiber membrane for hematocatharsis and a hollow-fiber membrane typ artificial kidney in which a hydrophilic polymer in the hollow-fiber membrane is less and a blood platelet is not adsorbed. The hollow-fiber membrane is prepared from a membrane-forming stock solution in which a hydrophilic polymer and a hydrophobic polymer are solvent and mixed in a common solvent, a hollow-fiber membrane for hematocatharsis in which the ratio of the hydrophilic polymer is used against the hydrophobic polymer on an outer surface of the hollow-fiber membrane is 5 to 25 %.

JP 2010-104984 discloses a polysulfone-based hollow-fiber membrane module of high performance which exhibits less adhesion of organic matter, protein, platelets and the like. The polysulfone-based hollow-fiber membrane module is incorporated with polysulfone-based hollow-fiber membranes. In the module, the ratio of measuring points is $\leq$10% wherein an average value of the ratio ($A_{co}/A_{cc}$) of the infrared absorption peak intensity ($A_{co}$) derived from an ester group C=O near 1,730 $cm^{-1}$ to the infrared absorption peak intensity ($A_{cc}$) derived from a benzene ring C=C of a polysulfone-based polymer near 1,580 $cm^{-1}$ on the functional layer surface near the end face and central part in the longitudinal direction of the polysulfone-based hollow-fiber membranes, is $\geq$0.005 and $\leq$0.001.

JP 4-325904 discloses an apparatus for drying a wet membrane formed of a plurality of fiber bundles at a time by means of micro-wave irradiation, capable of uniformly drying all the bundles. The apparatus for drying the hollow fiber membrane is characterized by comprising, in a vessel, a means of micro-wave irradiation, a means to carry the bundles fixed thereon into or out of the vessel, and a means to pass a liquid having a dielectric loss coefficient of 1 to 50 therethrough.

GB 1415597 discloses devices for the treatment of blood, in particular tubes having enzymes immobilized on the device. Such to increase their shelf-life and to protect the enzyme from leaching, the tube is on its inside with insoluble hydroxyethyl methacrylate polymer.

Summary of Invention

Problems To Be Solved By The Invention

However, conventional hollow-fiber membrane blood purification devices provided with a hollow-fiber membrane (for treating blood) with a fat-soluble vitamin fixed thereto are all wet type. Dry type devices are not present.

Recently, blood purification treatment has been gradually spread all over the world, and blood purification devices are mainly used at present in Japan and the United States and European countries and are also being used in a variety of regions such as China, India, Russia, Middle Eastern countries, South/Central American Countries and Southeastern Asian. The hollow-fiber membrane blood purification devices used in such a wide variety of countries are treated as medical equipment but states of control vary. At present, three-year storage stability under a normal temperature and normal humidity environment is recognized and guaranteed as general storage form; however, such guarantee is not sufficient.

For example, if a cargo container accommodating hollow-fiber membrane blood purification devices is exposed to direct sunlight in a container yard, the interior temperature of them is likely to rise to 60°C or more. Alternatively, it is conceivable that the devices are placed under unexpectedly high temperature environments for a long time when they are transported by trucks for a long time in large countries like the United States or transported by ships passing the tropical zone (equatorial line).

If a hollow-fiber membrane blood purification device with a fat-soluble vitamin fixed thereto as mentioned above

is exposed to such severe environments beyond expectation in the art, various properties, in particular, antioxidative performance, may possibly deteriorate.

As described above, a dry-type hollow-fiber membrane blood purification device provided with a hollow-fiber membrane with a fat-soluble vitamin fixed thereto, which has excellent antioxidative performance and light weight, are advantageous in view of distribution including transportation and storage; but are disadvantageous if exposed to severe environments as mentioned above, because antioxidative performance may deteriorate.

Then, an object of the present invention is to provide a dry-type hollow-fiber membrane blood purification device having excellent antioxidative performance and light weight, being excellent in view of distribution including transportation and storage and capable of stably maintaining antioxidative performance sufficient for practical use even it is placed under severe environments for a long time.

Means For Solving The Problems

The present inventors repeatedly conducted intensive studies with the view to attaining the above object. As a result, they found that a dry type hollow-fiber membrane blood purification device provided with a hollow-fiber membrane, which comprises a polysulfone-based resin and a polyvinyl pyrrolidone, to the surface of which a predetermined amount of fat-soluble vitamin is fixed, and an infrared spectrum of an ethanol extract of which has absorbance ratios of two peaks or shoulders present in a predetermined wave number region at predetermined values, is effective.

Based on the finding, they successfully obtained a hollow-fiber membrane blood purification device having excellent antioxidative performance and light weight, being excellent in view of distribution including transportation and storage and capable of stably maintaining excellent antioxidative performance under severe environments and completed the present invention.

More specifically, the present invention is as follows:

[1] A hollow-fiber membrane blood purification device provided with a hollow-fiber membrane comprising a polysulfone-based resin, a polyvinyl pyrrolidone and a fat-soluble vitamin E, wherein
the abundance of the fat-soluble vitamin E on all surfaces of the hollow-fiber membrane is 2 mg or more and 18 mg or less based on 1 g of the hollow-fiber membrane, whereby all surfaces includes the inner surface of the hollow-fiber in direct contact with blood, the outer surface and inner surface of pores of a porous portion of a membrane-thickness portion, and
when the hollow-fiber membrane is extracted with ethanol, the extract is dried and subjected to measurement of an infrared absorption spectrum (IR), a ratio of absorbance (Abs(A)) of peak A in the vicinity of 1720 to 1730 cm-1 to absorbance (Abs(B)) of peak B in the vicinity of 1660 to 1670 cm-1, (Abs (A)/Abs (B)), is 0.01 or more and 0.3 or less, and
wherein the moisture content of the hollow-fiber membrane is 0 mass% or more and 600 mass% or less, wherein the hollow-fiber membrane comprises a polymer having an ether group, wherein the polymer having the ester group comprises a polymer comprising any one selected from the group consisting of a methacrylate monomer, an acrylate monomer and an ester monomer of vinyl alcohol and a carboxylic acid, wherein the abundance of the fat-soluble vitamin E, the ratio of absorbance and the moisture content are determined as described later in the description.

[2] The hollow-fiber membrane blood purification device according to [1], wherein the abundance ratio of the polyvinyl pyrrolidone, which is the mass of polyvinylpyrrolidone based on the total mass of the polysulfone-based resin and the polyvinyl pyrrolidone, on the inner surface of the hollow-fiber membrane is 20 mass% or more and 90 mass% or less.

Advantageous Effects of Invention

According to the present invention, it is possible to obtain a hollow-fiber membrane blood purification device having high level blood compatibility, excellent antioxidative performance and antioxidative performance excellent in stability under severe environments.

Brief Description of Drawings

[Figure 1] Figure 1 shows one example of an infrared absorption spectrum of an extract obtained by extracting the hollow-fiber membrane of the hollow-fiber membrane blood purification device of the present embodiment with ethanol.

[Figure 2] Figure 2 shows another one example of an infrared absorption spectrum of an extract obtained by extracting the hollow-fiber membrane of the hollow-fiber membrane blood purification device of the present embodiment with ethanol.

[Figure 3] Figure 3 shows one example of an infrared absorption spectrum of an extract obtained by extracting the hollow-fiber membrane of the conventional hollow-fiber membrane blood purification device having no polymer having an ester group with ethanol.

Mode For Carrying Out The Invention

Now, an embodiment (hereinafter, referred to as "the present embodiment") for carrying out the invention will be more specifically described below.

The following embodiment is an example for explaining the present invention.

[Hollow-fiber membrane blood purification device]

The hollow-fiber membrane blood purification device according to the present embodiment is the hollow-fiber membrane blood purification device provided with the hollow-fiber membrane comprising a polysulfone-based resin, a polyvinyl pyrrolidone, a fat-soluble vitamin A and a polymer having an ester group, as described above.

The abundance of the fat-soluble vitamin on all surfaces of the hollow-fiber membrane is 2 mg or more and 18 mg or less based on 1 g of the hollow-fiber membrane.

When the hollow-fiber membrane is extracted with ethanol and the extract is dried and subjected to measurement of an infrared absorption spectrum (IR), the ratio of absorbance (Abs(A)) of peak A in the vicinity of 1720 to 1730 $cm^{-1}$ to absorbance (Abs(B)) of peak B in the vicinity of 1660 to 1670 $cm^{-1}$, (Abs(A)/Abs(B)), is 0.01 or more and 0.3 or less.

The moisture content of the hollow-fiber membrane is 0 mass% or more and 600 mass% or less.

Note that, in the specification, "peak A" and "peak B" each may include a bending portion of a curve in an infrared absorption spectrum, in other words, a shoulder portion.

(Hollow-fiber membrane)

The hollow-fiber membrane constituting the hollow-fiber membrane blood purification device according to the present embodiment comprises a polysulfone-based resin, a polyvinyl pyrrolidone, a fat-soluble vitamin and a polyvinyl pyrrolidone, a fat-soluble vitamin and a polymer having an ester group.

<Polysulfone-based resin>

The polysulfone-based resin (hereinafter, sometimes referred to as PSf), which is a general term expressing a polymer compound having a sulfone bond, is not particularly limited; for example, a polysulfone polymer having any one of repeat units represented by the following formulas (1) to (5) is mentioned wherein n is preferably an integer of 2 to 200, more preferably 80 to 140 and further preferably 92 to 136.

A bisphenol-based polysulfone polymer of the following formula (1) is commercially available under the trade name of "Udel" from Solvay Advanced Polymers, LLC., or under the trade name of "Ultrason" from BASF Corp.

The bisphenol-based polysulfone polymer is available in a plurality of types different in the degree of polymerization but is not particularly limited.

[Formula 1]

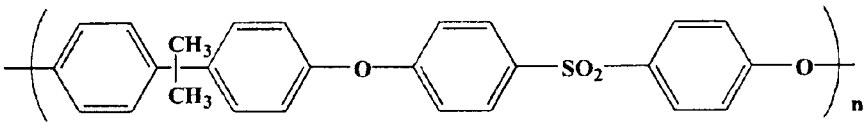

(1)

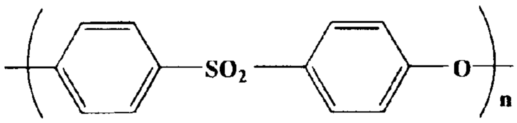

(2)

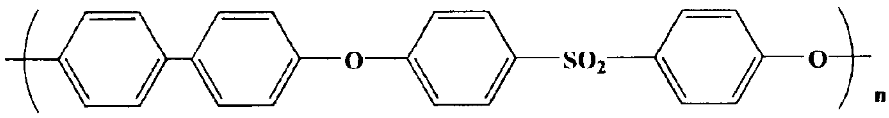

(3)

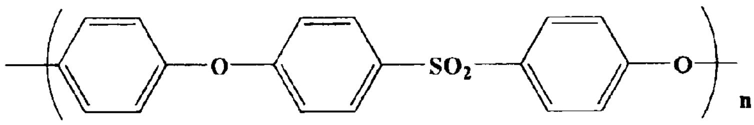

(4)

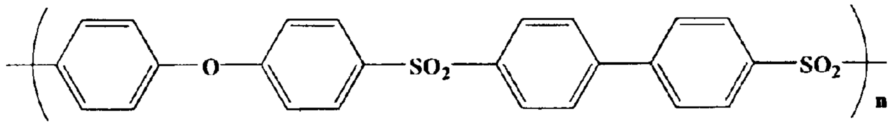

(5)

<Polyvinyl pyrrolidone>

The hollow-fiber membrane constituting the hollow-fiber membrane blood purification device according to the present embodiment comprises a polyvinylpyrrolidone (hereinafter, sometimes referred to as PVP) in consideration of spinning stability of a hollow-fiber membrane and affinity for the aforementioned PSf.

There are a plurality of PVPs different in the degree of polymerization. For example, K-15, K-30, and K-90 different in molecular weight are available under the trademark of "Plasdone" from ISP Ltd. Any of them can be used.

Note that PVP present on the inner surface of a hollow-fiber membrane enhances blood compatibility of the hollow-fiber membrane by forming a so-called diffuse layer in an aqueous medium such as blood. The diffuse layer refers to a highly hydrated PVP layer and covering over polysulfone present in the inner surface of a hollow-fiber membrane, thereby enhancing the blood compatibility. As described above, it is preferable that PVP forms a diffuse layer on the inner surface of a hollow-fiber membrane and has a large weight average molecular weight sufficient to cover over polysulfone.

It is difficult to accurately specify the weight average molecular weight of PVP present on the inner surface of a hollow-fiber membrane; however, since the weight average molecular weight of PVP eluted by circulating a solvent such as ethanol, which does not dissolve PSf but swells it, through a blood flow channel, is obtained by GPC (gel permeation chromatography), the molecular weight of PVP present on the inner surface of a hollow-fiber membrane can be obtained by relative comparison.

Note that, in the hollow-fiber membrane constituting the hollow-fiber membrane blood purification device according to the present embodiment, since a high molecular weight PVP not passing through dialysis membrane is localized on the inner surface, a routine method for measuring the weight average molecular weight of PVP by cutting a hollow-fiber membrane into pieces, soaking the pieces in a solvent and then extracting PVP is not proper.

In the hollow-fiber membrane constituting the hollow-fiber membrane blood purification device according to the present embodiment, when extraction is performed at 25°C for one hour by circulating ethanol (purity 99.5% or more) used as a solvent through a blood flow channel, the weight average molecular weight of the PVP extracted is preferably

60000 or more, since a diffuse layer can be easily formed on the inner surface of the hollow-fiber membrane and blood compatibility is improved, and more preferably 100000 or more. Note that the weight average molecular weight of the PVP extracted is a value for use in relative comparison. The actual average molecular weight of PVP on the inner surface of the hollow-fiber membrane is considered to be higher than this value.
This is because the higher the weight average molecular weight of PVP, the more frequently PVP gets tangled, with the result that it is difficult to completely extract PVP by the above method.

<Fat-soluble vitamin>

**According to the invention, the** fat-soluble vitamin **is vitamin E,** which is generally poorly soluble in water but soluble in alcohol and fat and oil.

Examples of vitamin E may include α-tocopherol, α-tocopherol acetate, α-tocopherol nicotinate, β-tocopherol, γ-tocopherol, and δ-tocopherol.

Particularly, α-tocopherol is preferably used since it has various physiological effects such as *in-vivo* antioxidative effect, a biomembrane stabilization effect and a platelet aggregation inhibitory effect.

The fat-soluble vitamin plays a role in mitigating oxidative stress observed in patients receiving dialysis for a long time, more specifically, plays a role in eliminating an oxidative stress causative substance, a peroxide, and in regaining *in-vivo* antioxidative effect.

In the hollow-fiber membrane constituting the hollow-fiber membrane blood purification device according to the present embodiment, the above effects are conceivably exerted by the presence of a fat-soluble vitamin over all surfaces of the hollow-fiber membrane.

The "all surfaces of the hollow-fiber membrane" include not only the inner surface of the hollow-fiber membrane in direct contact with blood but also the outer surface and inner surface of pores of a porous portion of a membrane-thickness portion. Of the blood components, hemocytes are only in contact with the inner surface; however, liquid components such as proteins, and peroxides such as active oxygen move in and out by diffusion through a membrane-thickness portion. Thus, all membrane surfaces including the surface of the porous portion and the outer surface contribute to the antioxidative effect.

Furthermore, a processing liquid (for example, a humectant used for leakage check, a preservation liquid and a dialysis fluid) and a gas contained in package bag in the case of a dry product are in contact with all surfaces including the outer surface. For the reason, the total amount of fat-soluble vitamin present on all surfaces of a hollow-fiber membrane becomes important.
In other words, the fat-soluble vitamin which is embedded in a membrane substrate and cannot be in contact with liquid is placed on the outside of "abundance of a fat-soluble vitamin on all surfaces of a hollow-fiber membrane" defined in the present embodiment.

In the hollow-fiber membrane blood purification device according to the present embodiment, the abundance of the fat-soluble vitamin on all surfaces of the hollow-fiber membrane is 2 mg or more and 18 mg or less based on 1 g of the hollow-fiber membrane.

To define the amount of fat-soluble vitamin present on all surfaces of a hollow-fiber membrane (including the porous membrane-thickness portion and the outer surface), the amount of fat-soluble vitamin based on weight of a hollow-fiber membrane is specified. Assuming that the amount of fat-soluble vitamin is defined based on the area of a hollow-fiber membrane (generally refers to the inner surface area), even if the areas of membranes are equal, if the thicknesses of the membranes differ, the total surface areas of them significantly differ, with the result that the total amounts of fat-soluble vitamin significantly vary. Thus, it is difficult to determine the amount of fat-soluble vitamin based on membrane area for obtaining a predetermined effect without fail.

In contrast, the porosity of a hollow-fiber membrane formed of a polysulfone-based resin as a base material is almost constant even if the thickness of the membrane changes. Thus, the range of abundance for obtaining an excellent effect can be defined if the volume or weight is used as a base.

Then, in the present embodiment, taking reproducibility of measurement seriously, the abundance of a fat-soluble vitamin on all surfaces of a hollow-fiber membrane is defined based on the weight of the hollow-fiber membrane.

In the hollow-fiber membrane blood purification device according to the present embodiment, as described above, the abundance of the fat-soluble vitamin on all surfaces of the hollow-fiber membrane is 2 mg or more and 18 mg or less based on 1 g of the hollow-fiber membrane, preferably 3 mg or more and 16 mg or less, and more preferably 4 mg or more and 15 mg or less.

Sufficient antioxidative performance of a hollow-fiber membrane for practical use can be obtained by setting the abundance of the fat-soluble vitamin to be 2 mg or more based on 1 g of the hollow-fiber membrane. In contrast, in view of retaining antioxidative performance and preventing excessive hydrophobicity of a hollow-fiber membrane, thereby obtaining sufficient blood compatibility for practical use, the abundance of the fat-soluble vitamin is set to be 18 mg or less based on 1 g of the hollow-fiber membrane.

Note that the abundance of the fat-soluble vitamin present on all surfaces of the hollow-fiber membrane constituting the hollow-fiber membrane blood purification device according to the present embodiment can be evaluated by extracting the fat-soluble vitamin of the hollow-fiber membrane of a hollow-fiber membrane blood purification device with an aqueous alcohol solution or an aqueous solution of a predetermined surfactant and then subjecting the extract to quantification by liquid chromatography.

At this time, it is necessary to extract the whole amount of fat-soluble vitamin present on all surfaces of the hollow-fiber membrane. Thus the extraction solvent must have sufficiently high dissolubility of the fat-soluble vitamin. However, if a polysulfone resin serving as a substrate of a hollow-fiber membrane is swollen, the fat-soluble vitamin embedded in the substrate of the hollow-fiber membrane is possibly extracted. Because of this, an aqueous surfactant solution, which is capable of sufficiently dissolving a fat-soluble vitamin without swelling the substrate of a hollow fiber membrane, is preferably used as an extraction solvent.

A method for measuring the abundance of a fat-soluble vitamin present on all surfaces of hollow-fiber membrane constituting a hollow-fiber membrane blood purification device will be specifically described below. Note that the method for measuring the abundance of a fat-soluble vitamin is not limited to the following examples. As long as the aforementioned object is attained, the collection amount, the concentration and quantity of an extraction solution, temperature, time, flow rate, measurement apparatus and the like can be appropriately modified.

First, a hollow-fiber membrane blood purification device is disassembled and a hollow-fiber membrane is taken out, washed with water and then dried.

Subsequently, e.g., 4 g of the hollow-fiber membrane dried above is weighed in a glass bottle, an aqueous surfactant solution, i.e., a 1 mass% aqueous solution of polyethylene glycol-t-octylphenyl ether (80 mL), was added thereto. Extraction is performed at room temperature for 60 minutes while applying ultrasonic vibration to obtain a fat-soluble vitamin extract.

A quantitative operation is performed by liquid chromatography. Using a calibration curve, which has been obtained from a peak area of a fat-soluble vitamin standard solution, the amount of fat-soluble vitamin of the extract is obtained.

A quantification method by means of liquid chromatography will be described below.

To a high performance liquid chromatographic apparatus (pump: JASCO PU-1580, detector: Shimadzu RID-6A, auto injector: Shimadzu SIL-6B, data processing: Tosoh GPC-8020, column oven: GL Sciences 556), a column (Shodex Asahipak ODP-506E packed column for HPLC) is equipped.
As a mobile phase, methanol (for high speed liquid chromatography) is supplied at a column temperature of 40°C and at a flow rate of e.g., 1 mL/min. From an absorption peak area of the UV region, the concentration of a fat-soluble vitamin is obtained. From the concentration, the weight (mg/g) of the surface fat-soluble vitamin contained in the hollow-fiber membrane is obtained assuming that the extraction efficiency is 100%.

Note that if a hollow-fiber membrane is stored under a severe environment such as a high temperature environment for a long time, the fat-soluble vitamin is partially inactivated.

The amount of fat-soluble vitamin present on all surfaces of the hollow-fiber membrane constituting the hollow-fiber membrane blood purification device according to the present embodiment is the amount of fat-soluble vitamin present on the hollow-fiber membrane surface including the amount of inactivated fat-soluble vitamin during storage under a severe environment.

In contrast, the "antioxidative capacity" described later corresponds to the abundance of fat-soluble vitamin not inactivated during storage under a severe environment, of the fat-soluble vitamin present on the surface of the hollow-fiber membrane.

As a method of adding a fat-soluble vitamin to a hollow-fiber membrane, various methods have been disclosed including a method of adding a fat-soluble vitamin to the entire hollow fiber membrane by adding a fat-soluble vitamin to an membrane-forming stock solution during formation of a membrane, a method of adding a fat-soluble vitamin to the inner surface of the hollow-fiber membrane by adding a fat-soluble vitamin and a surfactant to the bore liquid (Japanese Patent No. 4038583), and a method of attaching a fat-soluble vitamin to the inner surface of the hollow-fiber membrane by assembling a hollow-fiber membrane blood purification device, supplying a fat-soluble vitamin solution containing a fat-soluble vitamin and a solvent (for the fat-soluble vitamin) into a hollow portion of the hollow-fiber membrane (e.g., Japanese Patent Laid-Open No. 2006-296931). Any method of these and methods other than these may be employed.

<Polymer having an ester group>

The hollow-fiber membrane blood purification device according to the present embodiment comprises a polymer having an ester group.
**According to the invention, the** monomers constituting a polymer having the ester group **are selected from** a methacrylate, an acrylate and an ester between vinyl alcohol and a carboxylic acid.

Examples of the methacrylate and acrylate may include, but not limited to, esters of a fatty acid alcohol such

as methyl methacrylate, ethyl methacrylate and lauryl methacrylate; and esters of a fatty acid alcohol having a hydroxyl group such as hydroxyethyl methacrylate and hydroxypropyl methacrylate. Furthermore, these esters may have any type of functional group such as an amino group and a carboxylic acid group, a sulfonic acid group.

Furthermore, example of the ester between vinyl alcohol and a carboxylic acid may include, but not limited to, vinyl acetate and vinyl butyrate.

The polymer having an ester group may be a homopolymer constituted of a single monomer or a copolymer using a plurality of monomers selected from the aforementioned monomers.

The polymer having the ester group imparts stable antioxidative performance to a hollow-fiber membrane containing PSf, PVP and a fat-soluble vitamin under a severe environment.

The polymer having the ester group can be attached to a hollow-fiber membrane by dissolving it in a predetermined solvent to prepare a solution and passing the solution through the hollow-fiber membrane, or alternatively by previously dissolving it in the bore liquid (internal coagulant in hollow-fiber membrane) used in a step of producing a hollow-fiber membrane. Furthermore, the polymer can be attached to a hollow-fiber membrane by dissolving it in a dope used in a step of producing a hollow-fiber membrane. For the reason, the polymer having the ester group is preferably soluble in a solvent which cannot dissolve PSf, and preferably having an affinity for PVP present in the surface of a hollow-fiber membrane since stable antioxidative performance can be effectively obtained under a severe environment. Particularly, the effect of being soluble in a solvent not dissolving PSf and having affinity for PVP can be obtained by appropriately selecting a structure of a residue binding to a main chain via an ester bond or the monomer unit itself to be copolymerized.

Examples of the solvent dissolving the polymer having the ester group and not dissolving PSf may include, but not limited to, water, alcohols, hydrocarbons such as hexane and toluene, and chlorofluorocarbons ("CFC") or a CFC mixture, and a mixture between a PSf-soluble good solvent such as dimethylacetamide and a non-solvent such as water.

To control the solubility of the polymer to a solvent as mentioned above, for example, a methacrylate or acrylate having an alcohol or hydrophilic group such as an ionic functional group and a highly hydrophobic methacrylate or acrylate composed of an aliphatic or an aromatic group may be copolymerized in an appropriate ratio.

Further, it is preferable to copolymerize vinylpyrrolidone and ethylene glycol with the polymer having the ester group in view of controlling solubility of the polymer to a solvent as mentioned above.

In view of controlling the solubility of the polymer to the aforementioned solvent, a polymer having the ester group is preferably dissolved in water or an alcohol having up to 3 carbon atoms or a mixture of them, or preferably dissolved in an aqueous solution of dimethylacetamide (in the range of 0 mass% or more and 70 mass% or less).

The amount (abundance) of polymer having the ester group to be attached to the hollow-fiber membrane constituting the hollow-fiber membrane blood purification device according to the present embodiment can be defined by a relative ratio of the ester group and PVP in an extract of the hollow-fiber membrane.

A method of determining the relative ratio will be more specifically described below.

First, a hollow-fiber membrane blood purification device is disassembled, and a hollow-fiber membrane is taken out and dried at 40°C or less under vacuum until a constant weight is obtained. Subsequently, the dried hollow-fiber membrane is weighed in a glass bottle and extracted with ethanol (99.5%) added thereto at room temperature to 50°C or less for 60 minutes, while vibrating with ultrasonic wave. At this time, the bath ratio is controlled such that 20 mL of an extracting solvent is used based on the hollow-fiber membrane (1 g). The extract is filtered and ethanol is distilled away to obtain a dried product.

The relative ratio of the ester group and PVP is obtained based on the absorbance (Abs) ratio of the peak derived from the ester group based on the peak derived from PVP in the spectrum obtained by an infrared absorption method (IR).

A relative ratio is determined by IR as follows:

As an IR measurement apparatus, for example, Perkin Elmer Spotlight 200/Spectrum 100 can be used. A dried sample is placed on, for example, a barium fluoride plate, further pressed and thinly spread. Measurement is performed in accordance with a permeation method. From the obtained spectrum, the absorbance (Abs(A)) of peak A in the vicinity of 1720 to 1730 $cm^{-1}$ derived from the ester group and absorbance (Abs(B)) of peak B in the vicinity of 1660 to 1670 $cm^{-1}$ derived from polyvinylpyrrolidone are read out.

Note that if the positions of peaks are not clear, the absorbance at 1725 $cm^{-1}$ is regarded as Abs(A), and the absorbance at 1665 $cm^{-1}$ is regarded as Abs(B). The ratio (Abs(A)/Abs(B)) is calculated to obtain the relative ratio of the ester group and PVP.

The above (Abs(A)/Abs(B)) of the hollow-fiber membrane of the hollow-fiber membrane blood purification device according to the present embodiment is 0.01 or more and 0.3 or less in view of obtaining stable antioxidative capacity under a severe environment, as described later, preferably 0.02 or more and 0.3 or less, and more preferably 0.03 or more and 0.2 or less.

[Physical properties of a hollow-fiber membrane blood purification device]

<Moisture content of hollow-fiber membrane>

The hollow-fiber membrane blood purification device according to the present embodiment is a dry type. The dry type devices can be further divided into a dry type of narrow sense, in which the moisture content of a membrane is several percent or less and a dry type, in which the membrane is appropriately moistened with e.g., water or a humectant. The latter one is sometimes called as a semi-dry type distinguishably from the dry type of a narrow sense; however, since they have almost the same properties, they will be collectively referred to as a dry type in this specification.

Since the dry type devices are light compared to wet type devices and the interior liquid will not vigorously move even if impact is given, the hollow-fiber membrane is rarely broken. Besides this, they are rarely frozen at low temperature. Because of these features, the dry type devices are superior in view of distribution such as transportation and storage. In view of this, the moisture content of the hollow-fiber membrane constituting the hollow-fiber membrane blood purification device according to the present embodiment is 0 mass% or more and 600 mass% or less, preferably 0 mass% or more and 500 mass% or less and more preferably 0 mass% or more and 400 mass% or less.

Note that the moisture content of the whole hollow-fiber membrane can be calculated from weights before and after the hollow-fiber membrane is dried under vacuum until a constant weight is obtained, in accordance with the following expression. Actually, a trace amount of water is contained in the hollow-fiber membrane even if it is completely dried. Thus, the lower limit value of the moisture content is often 0.01 mass% or more and more specifically, 0.1 mass% or more.

[Expression 1]

$$\text{Moisture content} = \frac{(\text{Weight before dry}) - (\text{Weight after dry})}{(\text{Weight after dry})} \times 100 \quad (\text{Mass\%})$$

<Abundance ratio of PVP on the inner surface of a hollow-fiber membrane>

In the hollow-fiber membrane constituting the hollow-fiber membrane blood purification device according to the present embodiment, the abundance ratio of PVP on the inner surface of the hollow-fiber membrane is preferably 20 mass% or more and 90 mass% or less.

Herein, the abundance ratio of PVP on the inner surface of a hollow-fiber membrane refers to the mass ratio of PVP to the total mass of PSf and PVP in the uppermost surface layer portion in the interior of the hollow-fiber membrane (more specifically, the surface of the hollow-fiber membrane with which blood is brought into contact).

As a method for determining the abundance ratio of PVP on the inner surface of a hollow-fiber membrane, for example, a measuring method by X-ray photoelectron spectroscopy (XPS) is mentioned.

To describe it more specifically, the inner surface of hollow-fiber membrane was subjected to XPS measurement. Based on the peak strengths of atoms intrinsic to PSf and PVP, respectively, the number ratio of the atoms on the surface is obtained and then the weight ratio of both compounds is obtained based on the number ratio of the atoms. Based on the weight ratio, the abundance ratio can be calculated.

Specifically, the abundance ratio can be obtained from the number of nitrogen atoms (derived from PVP) and the number of sulfur atoms (derived from PSf) in the inner surface of the hollow-fiber membrane. Note that, on the hollow-fiber membrane constituting the hollow-fiber membrane blood purification device according to the present embodiment, there are a fat-soluble vitamin and a polymer having an ester group; however, the presence of them are ignored in measurement.

For example, when PSf is formed of the structural unit represented by the above formula (1), the abundance ratio of PVP on the inner surface of a hollow-fiber membrane can be obtained in accordance with the following expression.

[ Expression 2]

$$\text{Abundance ratio [\%] of polyvinylpyrrolidone} = \frac{\text{The number of nitrogen atom} \times 111}{\text{The number of nitrogen atom} \times 111 + \text{The number of sulfur atom} \times 442} \times 100 \quad \cdots (I)$$

In this formula, 111 is a formula weight of the repeat unit of PVP whereas 442 is a formula weight of the repeat

unit of PSf. In the case of other PSfs except PSf represented by the above formula (1), the abundance ratio can be obtained in the same manner using the formula weight of the repeat unit used as a resin skeleton.

By the presence of PVP on the inner surface of the hollow-fiber membrane, compatibility with blood can be improved. In view of this, the abundance ratio of PVP on the inner surface of a hollow-fiber membrane is preferably 20 mass% or more and 90 mass% or less, as mentioned above.

If the abundance ratio of PVP is 20 mass% or more, sufficient blood compatibility for practical use can be obtained. The abundance ratio of PVP is more preferably 27 mass% or more and further preferably 35 mass% or more.

In contrast, the upper limit of the abundance ratio of PVP on the inner surface of a hollow-fiber membrane is preferably 90 mass%. If the upper limit exceeds 90 mass%, even if priming of a hollow-fiber membrane blood purification device is performed, the air in the hollow portions is not removed. The amount of air called residual air tends to increase dramatically. If the amount of residual air is extremely high, there is a risk of feeding air into patient's body across a blood chamber.

Accordingly, the abundance ratio of PVP on the inner surface of a hollow-fiber membrane is preferably 90 mass% or less, more preferably 80 mass% or less and further preferably 50 mass% or less.

[Properties of a hollow-fiber membrane blood purification device]

(Antioxidative capacity of hollow-fiber membrane blood purification device)

In the embodiment, the antioxidative capacity of hollow-fiber membrane blood purification device can be obtained by the content of a fat-soluble vitamin on the hollow-fiber membrane.

The antioxidative capacity is, for example, the capacity to reduce ions by contacting a hollow fiber membrane with an aqueous solution of transition metal ions such as iron (III) ions and copper (II) ions and can be expressed by a numerical value obtained based on the capacity of pure $\alpha$-tocopherol to reduce ions.

For example, if $\alpha$-tocopherol is used as a fat-soluble vitamin, the antioxidative capacity of a hollow-fiber membrane per unit weight can be expressed based on the antioxidative capacity of pure $\alpha$-tocopherol, that is, in terms of a unit (mg/g HF), which is the amount (mg) of (pure) $\alpha$-tocopherol corresponding to the antioxidative capacity of the hollow-fiber membrane. For example, 4 mg/g HF means that hollow-fiber membrane (1 g) has an antioxidative capacity corresponding to antioxidative capacity of 4 mg of (pure) $\alpha$-tocopherol.

To obtain an antioxidative effect in blood purification treatment, the higher the antioxidative capacity, the more preferable. The effect can be obtained by at least 1 mg/g HF or more. Note that an antioxidative capacity of a conventional polysulfone-based hollow-fiber membrane blood purification device containing no fat-soluble vitamin is 0 mg/g HF.

(Handling of hollow-fiber membrane blood purification device)

In this embodiment, the properties of a dry-type hollow-fiber membrane blood purification device, in other words, features that the device has a light product weight compared to a wet type device and the hollow-fiber membrane is rarely broken because the liquid inside the device does not vigorously move even if impact is added and rarely frozen under low-temperature conditions, can be obtained by controlling the moisture content of a hollow-fiber membrane to a predetermined value.

The moisture content of the hollow-fiber membrane constituting the hollow-fiber membrane blood purification device according to the present embodiment may be 0 mass%, in other words, the membrane may be present in a completely dry state; however, the membrane generally contains a certain amount of water by absorbing water in the air. This is not an obstacle to obtain advantages of the invention.

In contrast, if the moisture content is too high, in other words, water freely moving within a container is present in a large amount, the container becomes too heavy to handle. In addition to this disadvantage, there is a risk that liquid damages a hollow-fiber membrane due to vibration and impact. Thus, a large content of water is not preferable.

For the reason, the moisture content of a hollow-fiber membrane is set at 600 mass% or less, preferably 500 mass% or less and more preferably 400 mass% or less.

Furthermore, assuming that the hollow-fiber membrane blood purification device according to the present embodiment is handled in cold weather regions, if the moisture content is excessively high, water is frozen, easily damage the follow-fiber membrane accompanying a structural change. Thus, high moisture content is not preferable. For the reason, the moisture content with respect to the whole hollow-fiber membrane is more preferably 50 mass% or less and further preferably 30 mass% or less.

(Stability of antioxidative performance of hollow-fiber membrane blood purification device under severe environment)

The antioxidative capacity of a hollow-fiber membrane blood purification device commercially available at

present and having a fat-soluble vitamin fixed thereto does not change (deteriorate) during storage at normal temperature and normal humidity for three years. However, deterioration by oxidation proceeds under a severe environment, with the result that the antioxidative capacity of a hollow-fiber membrane blood purification device may possibly reduce.

The stability of antioxidative capacity of the device with time by storage under a severe environment can be evaluated by an accelerated storage test.

For example, evaluation can be made by determining the antioxidative capacity of a module after heat treatment in an environment of 60°C for 6 days. As a result of the evaluation in this manner, it was found that the antioxidative capacity of a dry prototype of a hollow-fiber membrane blood purification device having a fat-soluble vitamin fixed thereto decreases by half (Comparative Example 1 of [Examples] described later). If an antioxidative capacity significantly decreases, a clinical effect exerted by the antioxidative capacity decreases. Thus, it is important to suppress the decrease of the antioxidative capacity.

As a result of intensive studies made by the present inventors, they surprisingly found that stability of the antioxidative capacity under a severe environment is significantly increased by adding a polymer having an ester group to a hollow-fiber membrane formed of PSf and PVP and having a predetermined amount of fat-soluble vitamin fixed thereto.

If a polymer having an ester group is added to a hollow-fiber membrane, a reduction rate of the antioxidative capacity after storage under a severe environment compared to that before the storage is preferably less than 50% and more preferably 25% or less.

The hollow-fiber membrane constituting the hollow-fiber membrane blood purification device according to the present embodiment contains a polysulfone-based resin, a polyvinyl pyrrolidone, a fat-soluble vitamin and a polymer having an ester group. The ester group has an effect of protecting a fat-soluble vitamin. If the abundance of a fat-soluble vitamin does not satisfy a predetermined amount, the effect of obtaining stable antioxidative capacity under a severe environment cannot be obtained. From this, it is considered that there is a certain interaction between the polymer having an ester group and a fat-soluble vitamin. In either case, the effect is apparent as described in Examples.

Specifically, the antioxidative performance of a hollow-fiber membrane does not reach a sufficient level for practical use, if the abundance of a fat-soluble vitamin on all surfaces of the hollow-fiber membrane does not satisfy a predetermined amount. In view of this, the abundance of a fat-soluble vitamin needs to be 2 mg or more based on 1 g of the hollow-fiber membrane, preferably 3 mg or more and further preferably 4 mg or more.

In contrast, if the abundance of a fat-soluble vitamin is extremely high, it is difficult to retain antioxidative capacity in the range (0.01 to 0.3) of the value (Abs(A)/Abs(B)) indicating the abundance of a polymer having an ester group of the embodiment. In addition, since hydrophobicity becomes excessively high, there is a risk of decreasing blood compatibility. For the reason, the abundance needs to be 18 mg or less and more preferably 15 mg or less.

In contrast, if the abundance of the polymer having the ester group is extremely low, the effect of obtaining stable antioxidative capacity under a severe environment cannot be obtained. The abundance of a polymer is 0.01 or more, more preferably 0.02 or more and further preferably 0.03 or more in terms of the aforementioned (Abs(A)/Abs(B)) value. If this value is extremely large, a relative concentration of PVP becomes insufficient, with the result that blood compatibility may deteriorate. Because of the risk, the abundance is 0.3 or less, more preferably 0.2 or less and further preferably 0.18 or less.

[Method for producing a hollow-fiber membrane blood purification device]

The hollow-fiber membrane blood purification device according to the present embodiment is obtained by first producing a hollow-fiber membrane, preparing a module using the hollow-fiber membrane, fixing a fat-soluble vitamin and a polymer having an ester group and subjecting to a sterilization treatment.

Fixation of a fat-soluble vitamin and a polymer having an ester group may be independently or simultaneously performed as described later. Alternatively fixation may be performed to a hollow-fiber membrane before the step of preparing a module.

(Step of producing a hollow-fiber membrane)

The hollow-fiber membrane can be produced by use of a dry-wet membrane formation technique known in the art.

First, PSf and PVP are dissolved in a common solvent to prepare a dope.

Examples of the common solvent may include, but not limited to, dimethylacetamide (hereinafter, referred to as DMAC), dimethylsulfoxide, N-methyl-2-pyrrolidone, dimethylformamide, sulfolane and dioxane and a mixture of 2 or more types of these solvents.

Note that an additive such as water may be added to the dope in order to control a pore diameter of a hollow-fiber membrane to a desired size.

The range of the concentration of PSf in the dope is not particularly limited as long as a membrane can be formed from

the dope and as long as the obtained hollow-fiber membrane can serve as a transparent membrane. For example, the concentration is preferably 5 to 35 mass% and more preferably 10 to 30 mass%. To attain high water permeability, the polymer concentration is preferably low, and further preferably 10 to 25 mass%. The concentration of PVP is controlled so as to obtain the mixing ratio of PVP to PSf preferably within the range of 27 mass% or less, more preferably 18 to 27 mass%, and further preferably 20 to 27 mass%. If the mixing ratio of PVP to PSf exceeds 27 mass%, an elution amount tends to increase. In contrast, if the mixing ratio is less than 18 mass%, the concentration of PVP in the membrane surface decreases and a sharp reduction of the leukocyte concentration in patient's blood, called Leukopenia symptom, is observed. For this reason, the mixing ratio less than 18 mass% is not preferable.

In the step of producing a hollow-fiber membrane, a tube-in-orifice spinneret is used. A dope and a bore liquid for coagulating the dope are simultaneously discharged into air from the orifice of the spinneret and from a tube, respectively.

As the bore liquid, water or a coagulant primarily containing water can be used. The composition and the like of the coagulant may be determined depending upon the permeability of a desired hollow-fiber membrane. Generally, a solution mixture of a solvent used in a dope and water is preferably used. For example, a 0 to 65 mass% aqueous DMAC solution is used. Furthermore, the abundance of PVP on the inner surface of a hollow-fiber membrane can be controlled by adding PVP to the bore liquid so as to obtain 0 to 2 mass%.

The dope discharged from the spinneret together with the bore liquid is allowed to run in the air and introduced in a coagulation bath primarily containing water and disposed in a lower portion of the spinneret and completely coagulated by soaking. Thereafter, a washing step or the like are performed. The resultant wet-state hollow-fiber membrane is wound by a wind-up machine to obtain a bundle of hollow-fiber membrane(s), which is further subjected to a dry treatment. Alternatively, after the above washing step, the hollow fiber membrane may be dried in a dryer to obtain a hollow fiber bundle.

In the hollow-fiber membrane thus obtained, the mass ratio of polyvinylpyrrolidone (abundance ratio of polyvinyl pyrrolidone) to the total mass of a polysulfone-based resin and polyvinylpyrrolidone in the inner surface of a hollow-fiber membrane is preferably 20 mass% or more and 90 mass% or less. If this range is satisfied, excellent anti-thrombogenicity and biocompatibility are obtained and deposition of e.g., proteins and platelets on the membrane surface is low.

(Step of producing a hollow-fiber membrane blood purification device)

The hollow-fiber membrane blood purification device according to the present embodiment can be produced by inserting a bundle of the aforementioned hollow-fiber membranes in a cylindrical container having an inlet and an outlet for a predetermined fluid, which is a fluid to be processed, injecting e.g., a polyurethane potting agent to both ends of the bundle to form a potting layer, sealing both ends, thereafter cutting off the remaining potting agent hardened to open an end surface, and attaching a header having an outlet and an inlet for fluid.

(Step of fixing fat-soluble vitamin to hollow-fiber membrane)

A step of fixing a fat-soluble vitamin to a hollow-fiber membrane can be carried out by various methods disclosed in the art, including a method of adding a fat-soluble vitamin to a membrane-forming stock solution during formation of a membrane, thereby adding the fat-soluble vitamin over the entire membrane; a method of adding a fat-soluble vitamin and a surfactant to a bore liquid, thereby depositing the fat-soluble vitamin onto the inner surface of a hollow-fiber membrane (for example, Japanese Patent No. 4038583 and Patent Document 2); and a method of supplying a fat-soluble vitamin solution containing a fat-soluble vitamin and a solvent (for the fat-soluble vitamin) into a hollow portion of the hollow-fiber membrane after a hollow-fiber membrane blood purification device is assembled, thereby depositing the fat-soluble vitamin onto the inner surface of hollow-fiber membrane (for example, Japanese Patent Laid open No. 2006-296931). However, any method including other methods may be used.

As a method for controlling the abundance of a fat-soluble vitamin on all surfaces of a hollow-fiber membrane to 2 mg or more and 18 mg or less based on 1 g of the hollow-fiber membrane, any method including methods disclosed in the aforementioned Patent Documents and other methods may be used.

Specifically, when a fat-soluble vitamin is added to a membrane-forming stock solution, it is preferable to control a concentration of the fat-soluble vitamin to be added to be 0.4 mass% to 2 mass% and more preferably to be 0.8 mass% to 1.5 mass%. If the concentration of the fat-soluble vitamin to be added is lower than 0.4 mass%, the amount of fat-soluble vitamin exposed from the surface of a hollow-fiber membrane is low, with the result that antioxidative capacity tends to be insufficient whereas if the concentration of the fat-soluble vitamin to be added exceeds 2 mass%, the strength of the hollow-fiber membrane sharply decreases, with the result that a risk of breakage of the hollow-fiber membrane may increase.

Furthermore, the hollow-fiber membrane thus formed is subjected to a heat treatment in a dry state at a temperature within a range of 100°C to 180°C and preferably 110 to 180°C, thereby a large amount of fat-soluble vitamin

can be localized on the surface even if the content of the fat-soluble vitamin on the hollow-fiber membrane is the same as that of other hollow-fiber membranes. If the temperature of the heat treatment is lower than 100°C, a localization effect is not exerted whereas if the temperature exceeds 180°C, the hollow-fiber membrane becomes soft and the permeability significantly changes. Thus such temperatures out of the range are not preferable.

When a fat-soluble vitamin is added to a bore liquid, the concentration of the fat-soluble vitamin is preferably controlled so as to fall within the range of 0.01 to 10 mass%, and more preferably within the range of 0.1 o 5 mass% and the amount of surfactant is added preferably in the ratio of 1/10 to 2 times with respect to the fat-soluble vitamin.

When a hollow-fiber membrane blood purification device assembled is coated with a fat-soluble vitamin solution, a coating solution is prepared by dissolving 0.1 to 2.0 mass% of the fat-soluble vitamin in a 50 to 80 mass% aqueous solution of an alcohol such as propanol, supplied and settled for a predetermined time, for example, 30 seconds to 60 minutes, preferably 1 to 10 minutes. After an excessive coating solution is blown away by air blow, the solvent may be removed by drying.

(Step of fixing a polymer having an ester group to hollow-fiber membrane)

A step of fixing a polymer having an ester group to a hollow-fiber membrane can be carried out by use of the same method as in fixing a fat-soluble vitamin.

Furthermore, this step may be carried out right after or prior to the step of fixing a fat-soluble vitamin.

A method for simultaneously fixing a fat-soluble vitamin and a polymer having an ester group to a hollow-fiber membrane, for example by preparing a solution mixture of them and coating the hollow-fiber membrane with the solution is preferable in view of production rationalization.

When a hollow-fiber membrane is coated with a solution, a solvent may be removed by drying with air or a gas such as nitrogen or drying under vacuum drying. A drying method is not particularly limited. Furthermore, the temperature in drying is not particularly limited.

The aforementioned (Abs(A)/Abs(B)) can be controlled to fall within the range of 0.01 or more and 0.3 or less by fixing a polymer having an ester group by the same method as in fixing the aforementioned fat-soluble vitamin.

For example, when a polymer having an ester group is added to a bore liquid, the polymer may be previously dissolved in the bore liquid in a concentration of preferably 50 to 20000 ppm and more preferably 500 to 2000 ppm.

When coating is performed by supplying a solution through a hollow portion of a hollow-fiber membrane, a coating solution having a concentration of preferably 50 to 20000 ppm and more preferably 500 to 2000 ppm is prepared and supplied. The coating solution is settled for a predetermined time, for example, 30 seconds to 60 minutes and preferably 1 to 10 minutes, and then excessive coating solution may be blown away by air blow and the solvent may be removed by drying.

(Step of sterilization treatment)

A sterilization treatment is applied to the aforementioned hollow-fiber membrane blood purification device.

As a sterilization method, either a radiation sterilization method or a vapor sterilization method or the like can be selected.

A hollow-fiber membrane containing a large amount of fat-soluble vitamin has a risk of causing a hollow fiber breakage if excessively heated. For the reason, a radiation sterilization method is preferable.

In the radiation sterilization method, e.g., an electron beam, gamma rays and X rays, can be used. Any one of them may be used.

The irradiation dose in the case of $\gamma$ rays and electron beam usually falls within the range of 5 to 50 kGy; however, irradiation is preferably performed within the range of 20 to 40 kGy.

By virtue of radiation sterilization under such conditions, PVP constituting a hollow-fiber membrane is partially crosslinked to suppress elution of PVP while maintaining satisfactory blood compatibility.

Examples

Now, the present invention will be more specifically described by way of Examples and Comparative Examples.

First, various measurement methods used in Examples will be described.

[Abundance of a fat-soluble vitamin on all surfaces of hollow-fiber membrane]

Abundance of a fat-soluble vitamin on all surfaces of hollow-fiber membrane was determined as follows.

A hollow-fiber membrane blood purification device was disassembled, and a hollow-fiber membrane was taken out and washed with water. After washing with water, the membrane was dried under vacuum at 40°C.

After dried, the hollow-fiber membrane (4 g) was weighed in a glass bottle. To this, 80 mL of a 1 mass% aqueous solution of Triton X-100 (for chemical use, manufactured by Kishida Chemical Co., Ltd.) was added to extract a fat-soluble vitamin while applying ultrasonic vibration at room temperature for 60 minutes.

A quantitative operation was performed by liquid chromatography. Using a calibration curve, which was prepared from a peak area of a fat-soluble vitamin standard solution, the amount of fat-soluble vitamin of the extract was obtained.

To describe more specifically, to a high performance liquid chromatographic apparatus (pump: JASCO PU-1580, detector: Shimadzu RID-6A, auto injector: Shimadzu SIL-6B, data processing: Tosoh GPC-8020, column oven: GL Sciences 556), a column (Shodex Asahipak ODP-506E packed column for HPLC) was equipped. Methanol for high speed liquid chromatography serving as a mobile phase was supplied at a column temperature of 40°C at a flow rate of 1 mL/min. The concentration of a fat-soluble vitamin is obtained from the area of an absorption peak in the UV region.

From the concentration, the mass (mg/g) of the fat-soluble vitamin present on all surfaces of the hollow-fiber membrane was obtained, assuming that the extraction efficiency is 100%. The mass of the fat-soluble vitamin present on all surfaces of the hollow-fiber membrane refers to the mass of the fat-soluble vitamin extracted from all surfaces of the hollow-fiber membrane.

Note that the fat-soluble vitamin partially oxidized by a sterilization treatment was included in the amount of fat-soluble vitamin present on all surfaces of the hollow-fiber membrane. Thus, in order to obtain the amount of partially oxidized fat-soluble vitamin, 50 kGy of radiation was previously applied to a fat-soluble vitamin to be used for preparing a calibration curve in air and an absorption peak of the partially oxidized fat-soluble vitamin was previously obtained. This was included in a peak group for use in area calculation. In this manner, the amount of partially oxidized fat-soluble vitamin was added in calculation.

[Measurement of index (Abs(A)/Abs(B)) for abundance ratio of a polymer having an ester group]

The index (Abs(A)/Abs(B)) for the abundance ratio of a polymer having an ester group was determined by the following method.

A hollow-fiber membrane blood purification device was disassembled and a hollow-fiber membrane was taken out and dried under vacuum at 40°C until a constant amount was obtained.

After dried, the hollow-fiber membrane (1 g) and 20 mL of ethanol (99.5%, manufactured by Wako Pure Chemical Industries, Ltd.) were weighed in a glass bottle.

Extraction was performed at room temperature (25°C) for one hour under ultrasonic vibration by use of SONICOR DSC-103TH manufactured by SONICOR INSTRUMENT CORPORATION as an ultrasonic vibrator. During extraction, the interior temperature of the vibrator increased up to about 40°C.

An extract was filtered and dried by distilling away ethanol by vacuum drying to obtain a dried product.

An IR measurement apparatus, Perkin Elmer Spotlight 200/Spectrum 100 was used.

A sample of the dried product was placed on a barium fluoride plate, further pressed and thinly spread. Absorbance was measured by a permeation method within a measurement range of 750 to 4000 $cm^{-1}$ and in a cumulated number of 16.

From the obtained spectrum, the absorbance (Abs(A)) of peak A in the vicinity of 1720 to 1730 $cm^{-1}$ derived from an ester group and the absorbance (Abs(B)) of peak B in the vicinity of 1660 to 1670 $cm^{-1}$ derived from polyvinylpyrrolidone were read out. Note that if the positions of peaks are not clear, the absorbance at 1725 $cm^{-1}$ was regarded as Abs(A), and the absorbance at 1665 $cm^{-1}$ was regarded as Abs(B).

A method of reading out Abs(A) and Abs(B) will be more specifically described referring to Figure 1, an example of the spectrum.

Note that in Figure 1 to Figure 3, the abscissa indicates a wave number whereas the ordinate indicates absorbance.

In Figure 1, focusing the range of 1500 to 1900 $cm^{-1}$ in the spectrum, a point showing the lowest absorbance in the vicinity of 1800 $cm^{-1}$ and a point showing the lowest absorbance in the vicinity of 1550 $cm^{-1}$ were connected by a linear line, which is defined as a base line (in Figure 1, shown by a broken line 1). Based on the base line thus defined, Abs(B) and Abs(A) were read out (indicated by a dash-dot line 2 and a dash-dot-dot line 3, respectively).

The ratio of (Abs(A)/Abs(B)) was calculated to obtain the relative ratio of the ester group and PVP.

Note that, in Figure 1, Abs(A)/Abs(B) = 0.3. In Figure 2, using a dash-dot line 2 and a dash-dot-dot line 4, Abs(B) and Abs(A) were read out, respectively, to obtain Abs(A)/Abs(B) = 0.01. Figure 3 shows an examples of Abs(A)/Abs(B) = 0. More specifically, Figure 1 and Figure 2 each show an example of a spectrum of an extract obtained by extracting the hollow-fiber membrane of the hollow-fiber membrane blood purification device according to the present embodiment with ethanol. In either one of the spectra, a peak or shoulder derived from an ester group was observed in the vicinity of 1720 to 1730 $cm^{-1}$.

Figure 3 shows an example of an absorption spectrum of an extract obtained by extracting the hollow-fiber membrane of a conventional hollow-fiber membrane blood purification device, containing no polymer having an ester

group with ethanol. In the spectrum, no peak derived from an ester group was observed in the vicinity of 1720 to 1730 $cm^{-1}$.

[Determination of the moisture content of a hollow-fiber membrane]

The moisture content of a hollow-fiber membrane was determined as follows.

A hollow-fiber membrane blood purification device was disassembled and a hollow-fiber membrane was taken out, dried under vacuum until the constant amount of hollow-fiber membrane was obtained. From the weight values before and after drying, a moisture content was determined in accordance with the following expression.

[Expression 3]

$$\text{Moisture content} = \frac{(\text{Weight before dry}) - (\text{Weight after dry})}{(\text{Weight after dry})} \times 100 \quad (\text{Mass\%})$$

[Abundance ratio of PVP on the inner surface of hollow-fiber membrane]

A hollow-fiber membrane blood processing device was disassembled and a hollow-fiber membrane was taken out.

The hollow-fiber membranes were tied with a string to prepare a bundle having about 50 filaments × about 20 cm. The bundle was soaked overnight in a vat filled with distilled water. Note that the distilled water was allowed to overflow from the vat by constantly supplying fresh distilled water.

The bundles of hollow-fiber membranes were taken out, cut into pieces of 5 cm in size, frozen by placing them in a freezer of -40°C and lyophilized overnight under a vacuum of about 40 to 53.3 Pa (0.3 to 0.4 torr). The dried hollow-fiber membrane was longitudinally cut and the inner surface was exposed. Several pieces were arranged on two-sided tape and used as samples. Measurement was performed by X-ray photoelectron spectroscopy instrument (ESCALAB250 manufactured by Thermo Fisher Scientific K.K.) under the following conditions.

Measurement conditions:

Excitation source: mono. AlK$\alpha$ 15 kV × 10 mA

Region taken up

Survey scan: 0 to 1,100 eV

Narrow scan: C1s, O1s, N1s, and S2p

Pass Energy: 100 eV

From the area strength of the Narrow Scan spectrum obtained, the concentrations of elements were obtained by use of relative sensitivity coefficients in the library of the apparatus and quantity calculation was made. The relative sensitivity coefficients used herein are C1s: 0.296, O1s: 0.711, S2p: 0.666, N1s: 0.477.

Now, explanation will be made by way of a case of a hollow-fiber membrane where a polysulfone represented by the above formula (1) was used as a polysulfone-based resin and a polyvinylpyrrolidone was used as a hydrophilic polymer.

S2p obtained by measurement is derived from polysulfone and N1s is derived from the polyvinyl pyrrolidone. The formula weight of a repeat unit of the polysulfone is 442 and the formula weight of a repeat unit of the polyvinylpyrrolidone is 111. Assuming that the element concentration of S2p is represented by S and the element concentration of N1s represented by N, the abundance ratio of polyvinylpyrrolidone in the inner surface of the hollow-fiber membrane was obtained by the following formula.

[Expression 4]

$$\frac{111 \times N}{(111 \times N) + (442 \times S)} \times 100 \quad (\text{Mass\%})$$

In Examples described later, each of the abundance ratios of polyvinylpyrrolidone in the inner surface of the hollow-fiber membrane was 20 mass% or more and 90 mass% or less.

[Antioxidative capacity immediately after production]

The antioxidative capacity of a hollow-fiber membrane blood purification device immediately after production was determined by the following method.
First, ferric chloride hexahydrate was dissolved in pure water to prepare a 0.3 w/v% aqueous solution (the amount (g) of solute in 100 mL of a solution).
A hollow-fiber membrane blood purification device was disassembled and a hollow-fiber membrane was taken out, washed with water and then dried at 40°C under vacuum. After dried, the hollow-fiber membrane (1 g) and an aqueous ferric chloride solution (20 mL) were weighed in a glass bottle, defoamed at 8000 Pa (60 mmHg) for 10 minutes and then incubated at 30°C for 4 hours while shaking (fat-soluble vitamin present on the hollow-fiber membrane surface reduces iron (III) ion to generate iron (II)).
The aqueous solution (2.6 mL) incubated was mixed with ethanol (0.7 mL) and a 0.5 w/v% aqueous 2,2'-bipyridyl ethanol solution (0.7 mL) separately prepared. The mixture was incubated at 30°C for 30 minutes while shaking (iron (II) and bipyridyl form a complex to emit color).
The absorbance of the colored solution at 520 nm was measured by a spectrometer.
Incubation, color reaction and absorbance measurement were performed in the same manner by use of an ethanol solution of the fat-soluble vitamin known in concentration in place of the hollow-fiber membrane to prepare a calibration curve. The antioxidative capacity provided by the hollow-fiber membrane (1 g) was obtained in terms of the corresponding weight of the fat-soluble vitamin (round off after the decimal point).
If the corresponding weight of the fat-soluble vitamin present on the hollow-fiber membrane surface based on 1 g of the hollow-fiber membrane was 1 mg or more, the antioxidative capacity was evaluated as being satisfactory (represented by O). If the corresponding weight of the fat-soluble vitamin was less than 1 mg, the antioxidative capacity was evaluated as being unsatisfactory (represented by X).

[Antioxidative capacity after storage under severe environment]

Hollow-fiber membrane blood purification devices (effective membrane area: 1.5 $m^2$) in Examples and Comparative Examples described later were treated with heat by storing them in a constant temperature vessel of 60°C for 6 days. Thereafter, the antioxidative capacities of the devices treated with heat were determined in the same manner as described in [antioxidative capacity immediately after production].
If the antioxidative capacity of a heat treated product is less than 1 mg or equal to or less than the half of the antioxidative capacity immediately after production, the stability of the antioxidative capacity was evaluated as being low (represented by X) and if not, the stability was evaluated as being high (represented by O).

[Hollow-fiber membrane blood purification device]

(Example 1)

A homogeneous dope was prepared from

PSf (P-1700 manufactured by Solvay Advanced Polymers, LLC.): 17 parts by mass
PVP (K-90 manufactured by ISP Ltd.): 4 parts by mass, and
Dimethylacetamide (hereinafter, DMAC): 79 parts by mass.

Herein, PSf represents a polysulfone-based resin and PVP represents polyvinyl pyrrolidone.
As the bore liquid, an aqueous 42 mass% DMAC solution was used and discharged together with a dope from a spinneret.
At this time, the amounts of dope and the bore liquid discharged were controlled so as to obtain a thickness of the dried membrane of 45 μm and an inner diameter of 185 μm.
The dope discharged was soaked in a coagulation bath consisting of water at 60°C provided at a level of 50 cm below, subjected to a coagulation step and a water-washing step (washing treatment with water) at a rate of 30 m/minute and then introduced into a dryer. After dried at 120°C for 4 minutes, the polysulfone-based hollow-fiber membrane was crimped and wound.
Subsequently, a plastic cylindrical container, which was designed so as to have an effective hollow-fiber membrane area of 1.5 $m^2$, was charged with a bundle formed of 10000 of hollow-fiber membranes wound, a urethane resin was applied to the both ends of the bundle to adhere them. Both end surfaces were cut to form open ends of the hollow-fiber membrane.

In an aqueous solution containing 57 parts by mass of 2-propanol (special grade, manufactured by Wako Pure Chemical Industries, Ltd.) and 43 parts by mass of distilled water (manufactured by Otsuka Pharmaceutical Co., Ltd.), poly(2-hydroxyethyl methacrylate) (average molecular weight of 20000, manufactured by SIGMA-ALDRICH, hereinafter referred to as p-HEMA) and $\alpha$-tocopherol (special grade, manufactured by Wako Pure Chemical Industries, Ltd.) were dissolved in concentrations of 0.1 mass% and 0.7 mass%, respectively, to obtain a homogeneous solution.

The solution (120 mL) was supplied from an open end into a hollow-fiber membrane and settled for 2 minutes and thereafter flashed with air of 0.3 MPa for 10 seconds. Subsequently, drying was performed for 4 hours while supplying dry nitrogen of 40°C. After drying, a header cap was equipped to both end portions.

After a stopper was provided to blood inlet- and outlet-side nozzles, $\gamma$ ray (25 kGy) was applied to obtain a hollow-fiber membrane blood purification device having an effective membrane area of 1.5 $m^2$.

The abundance of a fat-soluble vitamin ($\alpha$-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.1 and the moisture content was 0 mass%.

Various performances were measured and the results are shown in Table 1 below.

(Example 2)

In an aqueous solution containing 2-propanol (57 parts by mass) and distilled water (43 parts by mass), p-HEMA and $\alpha$-tocopherol were dissolved so as to obtain concentrations of 0.01 mass% and 0.7 mass%, respectively, to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin ($\alpha$-tocopherol) on a hollow-fiber membrane surface of the hollow-fiber membrane blood purification device was 5 mg based on 1 g of the hollow-fiber membrane. The (Abs(A)/Abs(B)) value measured by extracting a dried hollow-fiber membrane was 0.01 and the moisture content was 0 mass%.

Various performances were measured and the results are shown in Table 1 below.

(Example 3)

In an aqueous solution containing 2-propanol (57 parts by mass) and distilled water (43 parts by mass), p-HEMA and $\alpha$-tocopherol were dissolved so as to obtain concentrations of 0.2 mass% and 0.7 mass%, respectively, to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin ($\alpha$-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.3 and the moisture content was 0 mass%.

Various performances were measured and the results are shown in Table 1 below.

(Example 4)

In an aqueous solution containing 2-propanol (57 parts by mass) and distilled water (43 parts by mass), p-HEMA and $\alpha$-tocopherol were dissolved so as to obtain concentrations of 0.1 mass% and 0.3 mass%, respectively, to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin ($\alpha$-tocopherol) on a hollow-fiber membrane surface of the hollow-fiber membrane blood purification device was 2 mg based on 1 g of the hollow-fiber membrane. The (Abs(A)/Abs(B)) value measured by extracting the dried hollow-fiber membrane was 0.1 and the moisture content was 0 mass%.

Various performances were measured and the results are shown in Table 1 below.

(Example 5)

In an aqueous solution containing 2-propanol (57 parts by mass) and distilled water (43 parts by mass), p-HEMA and $\alpha$-tocopherol were dissolved so as to obtain concentrations of 0.1 mass% and 3 mass%, respectively to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin ($\alpha$-tocopherol) on a hollow-fiber membrane surface of the hollow-fiber membrane blood purification device was 18 mg based on 1 g of the hollow-fiber membrane. The (Abs(A)/Abs(B)) value measured by extracting the dried hollow-fiber membrane was 0.1 and the moisture content was 0 mass%.

Various performances were measured and the results are shown in Table 1 below.

(Example 6)

In an aqueous solution containing 2-propanol (57 parts by mass) and distilled water (43 parts by mass), poly(2-hydroxypropyl methacrylate) (manufactured by SIGMA-ALDRICH, hereinafter referred to as p-HPMA) and α-tocopherol were dissolved so as to obtain concentrations of 0.1 mass% and 0.7 mass%, respectively to obtain a homogeneous solution. The solution was supplied to a hollow-fiber membrane. A hollow-fiber membrane blood purification device was obtained by the same operation and under the same conditions as in Example 1 except this.
The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.1 and the moisture content was 0 mass%.
Various performances were measured and the results are shown in Table 1 below.

(Example 7)

Supplying a solution into a hollow-fiber membrane, air flash, drying by supplying dry nitrogen of 40°C for 4 hours, supplying 120 mL of distilled water from an open end to a hollow-fiber membrane, and flash with air (0.3 MPa) for 10 seconds were performed in the same manner as in Example 1. Subsequently, a header cap was equipped to the both end portions.
After a stopper was provided to blood inlet- and outlet-side nozzles, γ ray (25 kGy) was applied to obtain a hollow-fiber membrane blood purification device having an effective membrane area of 1.5 $m^2$.
The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.1 and the moisture content was 600 mass%.
Various performances were measured and the results are shown in Table 1 below.

(Example 8)

In an aqueous solution containing 2-propanol (57 parts by mass) and distilled water (43 parts by mass), Stylezer 2000 (copolymer of PVP, lauryl methacrylate and acrylic acid, manufactured by ISP) and α-tocopherol were dissolved so as to obtain concentrations of 0.7 mass% and 0.7 mass%, respectively to obtain a homogeneous solution. The solution was supplied to a hollow-fiber membrane. A hollow-fiber membrane blood purification device was obtained by the same operation and under the same conditions as in Example 1 except this.
The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.2 and the moisture content was 0 mass%.
Various performances were measured and the results are shown in Table 1 below.

(Example 9)

In an aqueous solution containing 2-propanol (57 parts by mass) and distilled water (43 parts by mass), p-HEMA, α-tocopherol, and (±)-α-tocopherol nicotinate (manufactured by Wako Pure Chemical Industries, Ltd.) were dissolved so as to obtain concentrations of 0.1 mass%, 0.35 mass% and 0.35 mass%, respectively to obtain a homogeneous solution. The solution was supplied to a hollow-fiber membrane. A hollow-fiber membrane blood purification device was obtained by the same operation and under the same conditions as in Example 1 except this.
The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.1 and the moisture content was 0 mass%.
Various performances were measured and the results are shown in Table 1 below.

(Comparative Example 1)

In an aqueous solution containing 2-propanol (57 parts by mass) and distilled water (43 parts by mass), α-tocopherol was dissolved so as to obtain concentration of 0.7 mass% to obtain a homogeneous solution. The solution was supplied to a hollow-fiber membrane. A hollow-fiber membrane blood purification device was obtained by the same operation and under the same conditions as in Example 1 except this.
The abundance of a fat-soluble vitamin (α-tocopherol) on a hollow-fiber membrane surface of the hollow-fiber

membrane blood purification device was 5 mg based on 1 g of the hollow-fiber membrane. The (Abs(A)/Abs(B)) value measured by extracting the dried hollow-fiber membrane was 0 and the moisture content was 0 mass%.

Various performances were measured and the results are shown in Table 2 below.

The antioxidative capacity after storage under severe environments significantly reduced and thus the object of the present invention could not be attained.

(Comparative Example 2)

In an aqueous solution containing 2-propanol (57 parts by mass) and distilled water (43 parts by mass), p-HEMA and α-tocopherol were dissolved so as to obtain concentrations of 0.005 mass% and 0.7 mass%, respectively to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0.005 and the moisture content was 0 mass%.

Various performances were measured and the results are shown in Table 2 below.

The antioxidative capacity after storage under severe environments significantly reduced and thus the object of the present invention could not be attained.

(Comparative Example 3)

In an aqueous solution containing 2-propanol (57 parts by mass) and distilled water (43 parts by mass), p-HEMA and α-tocopherol were dissolved so as to obtain concentrations of 0.1 mass% and 0.12 mass%, respectively to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin (α-tocopherol) on a hollow-fiber membrane surface of the hollow-fiber membrane blood purification device was 1 mg based on 1 g of the hollow-fiber membrane. The (Abs(A)/Abs(B)) value measured by extracting the dried hollow-fiber membrane was 0.1 and the moisture content was 0 mass%.

Various performances were measured and the results are shown in Table 2 below.

The antioxidative capacity after storage under severe environments significantly reduced and thus the object of the present invention could not be attained.

(Comparative Example 4)

PVP was used as a polymer having no ester group in place of a polymer having an ester group. In an aqueous solution containing 2-propanol (57 parts by mass) and distilled water (43 parts by mass), PVP and α-tocopherol were dissolved so as to obtain concentrations of 0.1 mass% and 0.7 mass%, respectively, to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0 and the moisture content was 0 mass%.

Various performances were measured and the results are shown in Table 2 below.

The antioxidative capacity after storage under severe environments significantly reduced and thus the object of the present invention could not be attained.

(Comparative Example 5)

Polyethylene Glycol 500,000 (manufactured by Wako Pure Chemical Industries, Ltd.; polyethylene glycol, hereinafter referred to as PEG) was used as a polymer having no ester group in place of a polymer having an ester group. In an aqueous solution containing 2-propanol (57 parts by mass) and distilled water (43 parts by mass), PEG and α-tocopherol were dissolved so as to obtain concentrations of 0.1 mass% and 0.7 mass%, respectively, to obtain a homogeneous solution. The solution was supplied to a hollow-fiber membrane. A hollow-fiber membrane blood purification device was obtained by the same operation and under the same conditions as in Example 1 except this.

The abundance of a fat-soluble vitamin (α-tocopherol) on a hollow-fiber membrane surface of the hollow-fiber membrane blood purification device was 5 mg based on 1 g of the hollow-fiber membrane. The (Abs(A)/Abs(B)) value measured by extracting the dried hollow-fiber membrane was 0 and the moisture content was 0 mass%.

Various performances were measured and the results are shown in Table 2 below.

The antioxidative capacity after storage under severe environments significantly reduced and thus the object of the present invention could not be attained.

[Table 1]

| | Example 1 | Example 2 | Example 3 | Example 4 | Example 5 | Example 6 | Example 7 | Example 8 | Example 9 |
|---|---|---|---|---|---|---|---|---|---|
| Additional polymer | p-HEMA | p-HEMA | p-HEMA | p-HEMA | p-HEMA | p-HPMA | p-HEMA | Stylezer 2000 | p-HEMA |
| Abs (A)/Abs (B) | 0.1 | 0.01 | 0.3 | 0.1 | 0.1 | 0.1 | 0.1 | 0.2 | 0.1 |
| Abundance of fat-soluble vitamin on hollow-fiber membrane surface (mg/gHF) | 5 | 5 | 5 | 2 | 18 | 5 | 5 | 5 | 5 |
| Moisture content (mass%) | 0 | 0 | 0 | 0 | 0 | 0 | 600 | 0 | 0 |
| Antioxidative capacity immediately after production (mg/gHF) | 4 (○) | 4 (○) | 4 (○) | 2 (○) | 17 (○) | 4 (○) | 4 (○) | 4 (○) | 2 (○) |
| Antioxidative capacity after storage under severe environment (mg/gHF) | 4 (○) | 4 (○) | 4 (○) | 2 (○) | 16 (○) | 4 (○) | 4 (○) | 4 (○) | 2 (○) |

[Table 2]

| | Comparative Example 1 | Comparative Example 2 | Comparative Example 3 | Comparative Example 4 | Comparative Example 5 |
|---|---|---|---|---|---|
| Additional polymer | None | p-HEMA | p-HEMA | PVP | PEG |
| Abs (A)/Abs (B) | 0 | 0.005 | 0.1 | 0 | 0 |
| Abundance of fat-soluble vitamin on hollow-fiber membrane surface (mg/gHF) | 5 | 5 | 1 | 5 | 5 |
| Moisture content (mass%) | 0 | 0 | 0 | 0 | 0 |
| Antioxidative capacity immediately after production (mg/gHF) | 4 (○) | 4 (○) | 1 (○) | 4 (○) | 4 (○) |
| Antioxidative capacity after storage under severe environment (mg/gHF) | 2 (×) | 2 (×) | 0 (×) | 2 (×) | 2 (×) |

In Table 1 and Table 2, the term "p-HEMA" refers to polyhydroxyethyl methacrylate;
The term "p-HPMA" refers to hydroxypropyl methacrylate;
The term "Stylezer 2000" refers to a copolymer of PVP, lauryl methacrylate and acrylic acid;
The term "PVP" refers to polyvinyl pyrrolidone; and
The term "PEG" refers to polyethylene glycol.

The hollow-fiber membrane blood purification devices of Examples 1 to 8 were each found to have satisfactory antioxidative performance for practical use and light weight (low moisture content) and have high antioxidative performance stability under severe environments.

Industrial Applicability

The hollow-fiber membrane blood purification device according to the present invention can endure storage not only under general storage conditions of sites where the device is mainly put in use at present in Japan and the United States and European countries but also under severe environments such as an unexpectedly high temperature environment for a long time in the case of transportation by truck in a large country for a long time and in the case of marine transportation by ship passing the tropical zone (equatorial line) and thus can be industrially applicable in various regions as a hollow-fiber membrane blood purification device by which blood extracorporeal circulation therapy is applied.

Reference Signs List

1 A base line (broken line), which is a linear line connecting a point showing the lowest absorbance in the vicinity of 1800 $cm^{-1}$ and a point showing the lowest absorbance in the vicinity of 1550 $cm^{-1}$
2 Absorbance (Abs(B)) (dash-dot line) of peak B derived from polyvinylpyrrolidone in the vicinity of 1660 to 1670 $cm^{-1}$
3 Absorbance (Abs(A)) (dash-dot-dot line) of peak A derived from an ester group in the vicinity of 1720 to 1730 $cm^{-1}$
4 Absorbance (Abs(A)) (height sandwiched between upper and lower arrows represented by dash-dot-dot lines) of peak A derived from an ester group in the vicinity of 1720 to 1730 $cm^{-1}$

**Claims**

1. A hollow-fiber membrane blood purification device provided with a hollow-fiber membrane comprising a polysulfone-based resin, a polyvinyl pyrrolidone and a fat-soluble vitamin E, wherein
the abundance of the fat-soluble vitamin E on all surfaces of the hollow-fiber membrane is 2 mg or more and 18 mg or less based on 1 g of the hollow-fiber membrane, whereby all surfaces includes the inner surface of the hollow-fiber in direct contact with blood, the outer surface and inner surface of pores of a porous portion of a membrane-thickness portion, and
wherein the moisture content of the hollow-fiber membrane is 0 mass% or more and 600 mass% or less,
**characterized in that** the hollow-fiber membrane comprises a polymer having an ester group, wherein the polymer having the ester group comprises a polymer comprising any one selected from the group consisting of a methacrylate monomer, an acrylate monomer and an ester monomer of vinyl alcohol and a carboxylic acid, and
when the hollow-fiber membrane is extracted with ethanol, the extract is dried and subjected to measurement of an infrared absorption spectrum (IR), a ratio of absorbance (Abs(A)) of peak A in the vicinity of 1720 to 1730 cm-1 to absorbance (Abs(B)) of peak B in the vicinity of 1660 to 1670 cm-1, (Abs(A)/Abs(B)), is 0.01 or more and 0.3 or less, wherein the abundance of the fat-soluble vitamin E, the ratio of absorbance and the moisture content are determined as described in the description.

2. The hollow-fiber membrane blood purification device according to claim 1, wherein the abundance ratio of the polyvinyl pyrrolidone, which is the mass of polyvinylpyrrolidone based on the total mass of the polysulfone-based resin and the polyvinyl pyrrolidone, on the inner surface of the hollow-fiber membrane is 20 mass% or more and 90 mass% or less.

## Patentansprüche

1. Hohlfasermembran-Blutreinigungsvorrichtung, die mit einer Hohlfasermembran versehen ist, welche ein Harz auf Polysulfonbasis, ein Polyvinylpyrrolidon und ein fettlösliches Vitamin E umfasst, wobei die Konzentration des fettlöslichen Vitamins E auf allen Oberflächen der Hohlfasermembran 2 mg oder mehr und 18 mg oder weniger beträgt, bezogen auf 1 g der Hohlfasermembran, wobei "alle Oberflächen" die innere Oberfläche der Hohlfaser in direktem Kontakt mit Blut, die äußere Oberfläche und die innere Oberfläche von Poren eines porösen Teils eines Membrandickenteils umfasst und wobei der Feuchtigkeitsgehalt der Hohlfasermembran 0 Massen-% oder mehr und 600 Massen-% oder weniger beträgt, **dadurch gekennzeichnet, dass** die Hohlfasermembran ein Polymer mit einer Estergruppe umfasst, wobei das Polymer mit der Estergruppe ein Polymer umfasst, das ein beliebiges umfasst, das aus der Gruppe ausgewählt ist, die aus einem Methacrylatmonomer, einem Acrylatmonomer und einem Estermonomer von Vinylalkohol und einer Carbonsäure besteht; und
wenn die Hohlfasermembran mit Ethanol extrahiert wird, der Extrakt getrocknet und einer Messung eines Infrarotabsorptionsspektrums (IR) unterzogen wird, dann beträgt das Verhältnis der Extinktion (Abs(A)) von Peak A in der Nähe von 1720 bis 1730 $cm^{-1}$ zu der Extinktion (Abs(B)) von Peak B in der Nähe von 1660 bis 1670 $cm^{-1}$ (Abs(A)/Abs(B)) 0,01 oder mehr und 0,3 oder weniger, wobei die Konzentration des fettlöslichen Vitamins E, das Verhältnis der Extinktionen und der Feuchtigkeitsgehalt so bestimmt werden, wie es in der Beschreibung beschrieben ist.

2. Hohlfasermembran-Blutreinigungsvorrichtung gemäß Anspruch 1, wobei das Konzentrationsverhältnis des Polyvinylpyrrolidons, d.h. die Masse des Polyvinylpyrrolidons, bezogen auf die Gesamtmasse des Harzes auf Polysulfonbasis und des Polyvinylpyrrolidons, auf der inneren Oberfläche der Hohlfasermembran 20 Massen-% oder mehr und 90 Massen-% oder weniger beträgt.

## Revendications

1. Dispositif de purification du sang à membrane en fibre creuse muni d’une membrane en fibre creuse comprenant une résine à base de polysulfone, une polyvinylpyrrolidone et une vitamine E soluble dans les graisses, où
l’abondance de la vitamine E soluble dans les graisses sur toutes les surfaces de la membrane en fibre creuse est 2 mg ou plus et 18 mg ou moins sur la base de 1 g de la membrane en fibre creuse, où toutes les surfaces incluent la surface interne de la fibre creuse en contact direct avec le sang, la surface externe et la surface interne de pores d’une partie poreuse d’une partie d’épaisseur de la membrane, et
où la teneur en humidité de la membrane en fibre creuse est 0 % en masse ou plus et 600 % en masse ou moins,
**caractérisé en ce que** la membrane en fibre creuse comprend un polymère ayant un groupe ester, où le polymère ayant le groupe ester comprend un polymère comprenant l’un quelconque choisi dans le groupe consistant en un monomère méthacrylate, un monomère acrylate et un monomère ester d’alcool vinylique et d’un acide carboxylique,
et quand la membrane en fibre creuse est soumise à une extraction avec de l’éthanol, l’extrait est séché et soumis à une mesure d’un spectre d’absorption infrarouge (IR), un rapport de l’absorbance (Abs(A)) du pic A au voisinage de 1720 à 1730 $cm^{-1}$ à l’absorbance (Abs(B)) du pic B au voisinage de 1660 à 1670 $cm^{-1}$, (Abs(A))/(Abs(B)), est

0,01 ou plus et 0,3 ou moins,
où l'abondance de la vitamine E soluble dans les graisses, le rapport d'absorbance et la teneur en humidité sont déterminés comme décrit dans la description.

**2.** Dispositif de purification du sang à membrane en fibre creuse selon la revendication 1, où le rapport d'abondance de la polyvinylpyrrolidone, qui est la masse de polyvinylpyrrolidone sur la base de la masse totale de la résine à base de polysulfone et de la polyvinylpyrrolidone, sur la surface interne de la membrane en fibre creuse est 20 % en masse ou plus et 90 % en masse ou moins.

FIG. 1

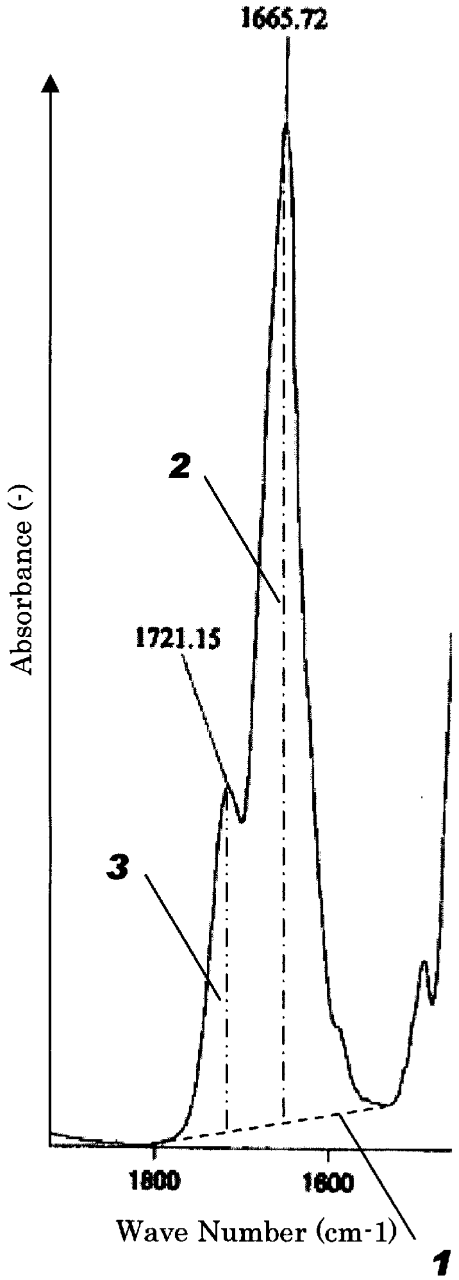

FIG. 2

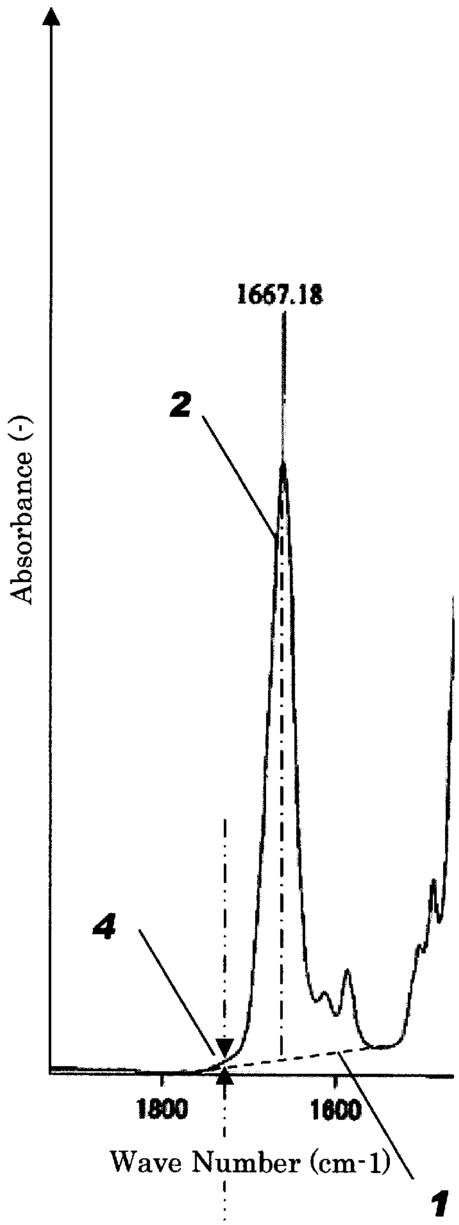

FIG. 3

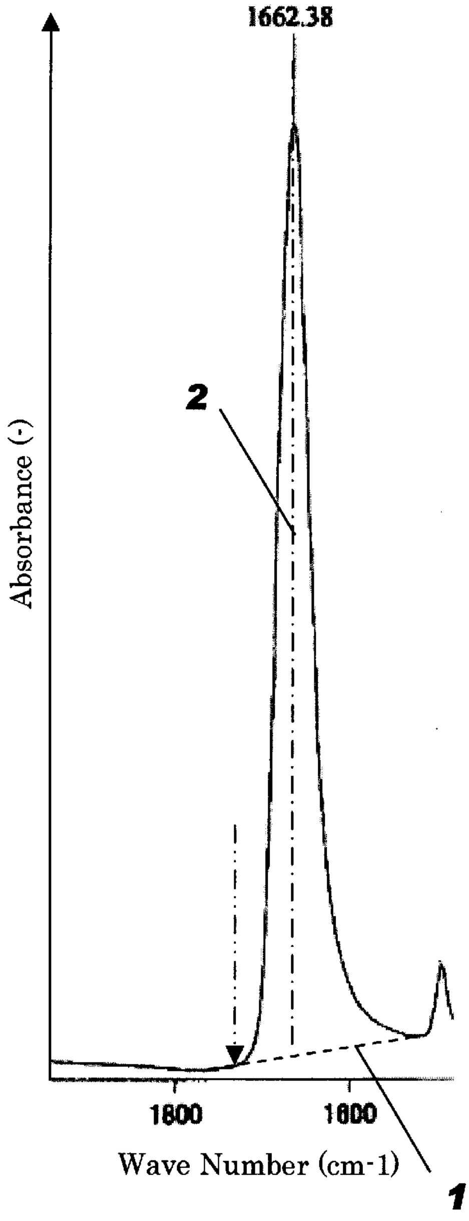

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H7178166 B
- JP 2009022635 A
- EP 2151273 A
- EP 2286902 A
- JP 4190079 A
- JP 2010104984 A
- JP 4325904 A
- GB 1415597 A
- JP 4038583 B
- JP 2006296931 A

### Non-patent literature cited in the description

- **ANDRULLI S.** *Nephron Clin Pract.,* 2010, vol. 115, c82-89
- **PANICHI V et al.** *Blood Purification.,* 2011, vol. 32, 7-14